(19) Europäisches Patentamt
European Patent Office
Office européen des brevets

(11) Veröffentlichungsnummer: **0 001 597**
**B1**

(12) **EUROPÄISCHE PATENTSCHRIFT**

(45) Veröffentlichungstag der Patentschrift: **21.10.81**

(21) Anmeldenummer: **78101081.4**

(22) Anmeldetag: **06.10.78**

(51) Int. Cl.³: **G 03 C 1/72**, G 03 F 7/08, C 08 K 5/34

(54) **Unter der Einwirkung von Licht härtbare, Bis-Azidophthalimidyl-Derivate enthaltende Stoffgemische.**

(30) Priorität: **14.10.77 CH 12581/77**

(43) Veröffentlichungstag der Anmeldung:
**02.05.79 Patentblatt 79/9**

(45) Bekanntmachung des Hinweises auf die Patenterteilung:
**21.10.81 Patentblatt 81/42**

(84) Benannte Vertragsstaaten:
**CH DE FR GB NL SE**

(56) Entgegenhaltungen:
**FR - A - 2 238 953**

**PHOTOGRAPHIC SCIENCE AND ENGINEERING, 17, 390-3 (1973)**
***Seite 391***

(73) Patentinhaber: **CIBA-GEIGY AG**
**Patentabteilung Postfach**
**CH-4002 Basel (CH)**

(72) Erfinder: **Zweifel, Hans, Dr.**
**Lothringerstrasse 93**
**CH-4056 Basel (CH)**
Erfinder: **Kvita, Vratislav, Dr.**
**St. Jakobstrasse 2**
**CH-4132 Muttenz (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 597 B1

Unter der Einwirkung von Licht härtbare, Bis-Azidophthalimidyl-Derivate enthaltende Stoffgemische

Die Erfindung betrifft neue unter der Einwirkung von Licht härtbare, Bis-Azidophthalimidyl-Derivate enthaltende Stoffgemische sowie deren Verwendung zur Vernetzung unter der Einwirkung von Licht, besonders zum Erzeugen von Abbildungen.

Aus der Literatur ist bekannt, dass sich Azido-Phenylderivate, wie 4,4'-Diazidostilben, das Dinatriumsalz von 4,4'-Diazidostilben-2,2'-disulfonsäure, 4,4'-Diazidobenzophenon, 4,4'-Diazidochalkon, 2,6-Di-(4'-Azidobenzal)-cyclohexanon, 2,6-Di-(4'-Azidobenzal)-4-methylcyclohexanon, 4,4'-Diazidobenzalaceton und Bis-(4'-Azidocinnamyliden)-cyclopentanon, als Sensibsilsatoren für Polymere für phototechnische Zwecke, z.B. sog. Photoresists, oder als lichtempfindliche Komponente in Kolloidschichten, wie Gelatine oder Kasein, zur Erzeugung von sogenannten Gerbbildern in der Photographie oder bei Verfahren in der Reproduktionstechnik eignen (vgl. z.B. US Patentschriften 2.852.379, 2.940.853 und 3.749.713, deutsche Patentschrift 752.852, russische Patentschrift 503.855 und britische Patentschrift 892.811).

Diese vorbekannten Azido-Phenylderivate sind insofern nachteilig, als die thermische Stabilität und die Oxidationsbeständigkeit derselben oder von Gemischen, die dieselben enthalten, zu wünschen übrig lassen.

Aufgabe der Erfindung war daher die Bereitstellung von unter der Einwirkung von Licht härtbaren Systemen mit verbesserter thermischer Stabilität und Oxidationsbeständigkeit bei gleich guter Lichtempfindlichkeit wie die von Stoffgemischen, welche die oben angeführten bekannten Substanzen enthalten.

Gegenstand der Erfindung sind somit unter der Einwirkung von Licht härtbare (vernetzbare) Stoffgemische, die mindestens eine gegebenenfalls härtbare polymere Verbindung und mindestens eine Verbindung der Formel I

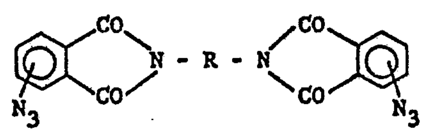

(I)

enthalten, worin

R unsubstituiertes oder substituiertes Alkylen mit 2—12 C-Atomen, unsubstituiertes oder substituiertes Phenylen, Naphthylen, Biphenylen, Cyclohexylen, Dicyclohexylmethan oder eine unsubstituierte oder substituierte Gruppe

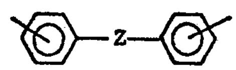

und

Z —O—, —S—, —$SO_2$—, —$CH_2$—, —CO—, $\underset{|}{\text{CH}}$— mit $CH_3$ (—CH($CH_3$)—) oder —C($CH_3$)$_2$— bedeuten,

wobei das Gewichtsverhältnis von polymerer Verbindung zu Verbindung der Formel I 9:1 bis 1:4 beträgt.

Durch R dargestellte Alkylengruppen können geradkettig oder verzweigt und gegebenenfalls substituiert sein, z.B. durch eine oder mehrere Phenylgruppen, Cycloalkylgruppen mit 5—8 C-Atomen oder Aralkylgruppen mit 7 oder 8 C-Atomen. Unter den substituierten Alkylengruppen R sind solche bevorzugt, die durch eine oder zwei Phenylgruppen oder durch eine oder zwei definitionsgemässe Cycloalkyl- oder Aralkylgruppen, wie die Cyclopentyl-, Cyclohexyl-, Cyclooctyl- oder die Benzylgruppe, substituiert sind.

Beispiele derartiger Alkylengruppen R sind: die 1,2-Aethylen-, 1,3- oder 1,2-Propylen-, 1,4-oder 1,3-Butylen-, Pentamethylen-, Hexamethylen-, 2-Methyl-4-dimethylhexan-, 2-Dimethyl-4-methylhexan-, 1,10-Dicyclohexyl- und 1,10-Dicyclooctyldecan-, 1,10-Di-isopropyldecan-, 1,1,10,10-Tetramethyldecan-, 1,10-Diäthyl-1,10-dimethyldecan-, Octamethylen-, Decamethylen- und Dodecamethylengruppe.

Bevorzugt sind unsubstituierte geradkettige oder verzweigte Alkylengruppen mit 2—12 und insbesondere 2—10 C-Atomen.

Durch R dargestellte Phenylen-, Naphthylen-, Biphenylen-, Cyclohexylen- oder Dicyclohexylmethangruppen und Gruppen R

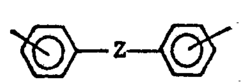

können ebenfalls substituiert sein, z.B. durch Alkylgruppen mit 1—4 C-Atomen, —OH—, —$COO^-M^{\pm}$—, oder —$SO_3^-M^+$-Gruppen, wobei

$M^+$ ein Wasserstoffion, ein Alkalimetallkation, das Pyridiniumkation oder die Gruppe

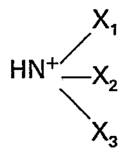

bedeutet, in der

$X_1$ und $X_2$ unabhängig voneinander gleich Wasserstoff oder Alkyl mit 1—12 C-Atomen und $X_3$ Wasserstoff, Alkyl mit 1—12 C-Atomen oder Benzyl sind. Alkylgruppen $X_1$, $X_2$ und $X_3$ können geradkettig oder verzweigt sein. Vorzugsweise handelt es sich um geradkettige Alkylgruppen mit 1—4 C-Atomen. Die genannten Gruppen R können an jedem Ring mehrere Substituenten der erwähnten Art aufweisen, sind aber mit Vorteil pro Ring nur durch eine der genannten Gruppen substituiert. Als Alkylsubstituenten kommen besonders Methyl und Aethyl in Betracht. Stellt $M^+$ eine Gruppe

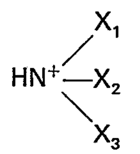

dar, so bedeuten $X_1$ und $X_2$ bevorzugt Alkylgruppen mit 1—4 C-Atomen und $X_3$ eine Alkylgruppe mit 1—4 C-Atomen oder die Benzylgruppe.

Bevorzugte $—COO^-M^+$- oder $—SO_3^-MN^+$-Substituenten sind solche, worin $M^+$ ein Wasserstoffion, ein Alkalimetallkation, ein Benzyldialkyl- oder Trialkylammoniumkation mit je 1—4 C-Atomen in den Alkylteilen darstellt. Besonders bevorzugt bedeutet $M^+$ ein Wasserstoffion, ein Natrium- oder Kaliumkation.

Phenylen-, Naphthylen-, Biphenylen-, Cyclohexylen- und Dicyclohexylmethangruppen oder Gruppen

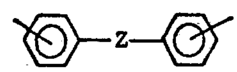

sind bevorzugt unsubstituiert. Bevorzugte Brückenglieder Z sind —O—, $—SO_2—$ und $—CH_2—$.

Die $N_3$-Gruppen sind bevorzugt je in 3-Stellung an den Benzolring gebunden.

Bevorzugt sind Stoffgemische mit einem Gewichtsverhältnis von polymerer Verbindung zu Verbindung der Formel I von 9:1 bis 1:1. Die erfindungsgemässen Stoffgemische können auch mehrere verschiedene Verbindungen der Formel I enthalten. Besonders bevorzugt sind Stoffgemische, die eine Verbindung der Formel I enthalten, worin R unsubstituiertes geradkettiges oder verzweigtes Alkylen mit 2—10 C-Atomen, unsubstituiertes Phenylen, oder einen unsubstituierten Diphenyläther-, Diphenylmethan- oder Diphenylsulfonrest bedeuten.

Als polymere Verbindungen, die sich unter der Einwirkung von Licht mit den Bis-Azidophthalimidylderivaten der Formel I vernetzen bzw. härten lassen, können an sich beliebige bekannte synthetische oder natürliche Polymere eingesetzt werden.

Beispiele geeigneter Polymere sind: Polyester, Polyesteramide, Polyamide, Polyamidsäuren, Polyamid-amidsäuren, Polyimide, Polyamid-imide, Polyäther, Polyamine, Polyimine, Polyurethane, Polyharnstoffe, Polyurethan-harnstoffe, Polycarbonate, Phenol-Formaldehyd-Polykondensate, Polysaccharide, Gelatine oder Polymere, die durch Homo- oder Copolymerisation von reaktive C=C-Doppelbindungen enthaltenden Monomeren erhalten werden.

Im folgenden werden einige bevorzugte Klassen von Polymeren angeführt.

*1. Polyamide, Polyamidsäuren, Polyamid-amidsäuren, Polyester und Polyesteramide,* die gleiche oder verschiedene wiederkehrende Strukturelemente der Formel II oder III

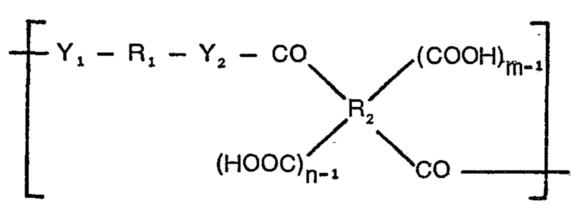

(II)

oder

$$-\!\!\left[NH - R_3 - CO\right]\!\!- \quad \text{aufweisen,}$$

(III)

und die entsprechenden cyclisierten Derivate (Polyimide und Polyamidimide);

2. *Polyurethane, Polyharnstoffe und Polyurethan-harnstoffe,* die gleiche oder verschiedene wiederkehrende Strukturelemente der Formel IV

$-[CO-NH-R_2-NH-CO-Y_1-R_1-Y_2$ (IV) aufweisen;

3. *Polyamine,* die gleiche oder verschiedene wiederkehrende Strukturelemente der Formel V oder VI

$$-[NH-R_1-NH-R_2]- \quad (V)$$

oder

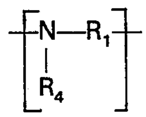

(VI)

aufweisen;

4. *Polycarbonate,* die gleiche oder verschiedene wiederkehrende Strukturelemente der Formel VII

$$-[O-R_2-O-CO]- \quad (VII)$$

aufweisen;

5. *Polyäther,* die wiederkehrende Strukturelemente der Formeln VIII, IX oder X

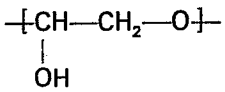

(VIII),

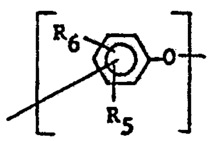

(IX)

oder

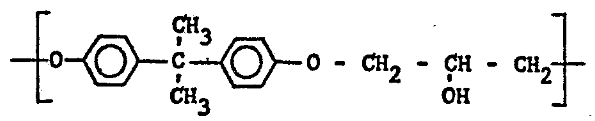

(X)

aufweisen;

6. *Phenol-Formaldehyd-Polykondensate* (Novolake), die wiederkehrende Strukturelemente der Formel XI

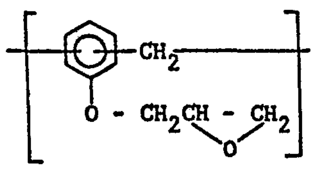

(XI)

aufweisen;

7. *Homo- oder Copolymere aus äthylenisch ungesättigten Monomeren* mit gleichen oder verschiedenen wiederkehrenden Strukturelementen der Formel XII

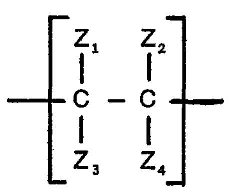

(XII);

8. *Cyclisierte Isoprenpolymere,*

In den obigen Formeln II bis XII bedeuten:

m und n unabhängig voneinander die Zahl 1 oder 2,

$Y_1$ und $Y_2$ unabhängig voneinander —O— oder —NH—,

$R_1$ einen aliphatischen Rest mit mindestens 2 C-Atomen, einen cycloaliphatischen, araliphatischen, carbocyclisch-aromatischen oder heterocyclisch-aromatischen Rest,

$R_2$ einen aliphatischen Rest mit mindestens 2 C-Atomen, einen cycloaliphatischen, carbocyclisch-aromatischen oder heterocyclisch-aromatischen Rest,

$R_3$ einen aliphatischen Rest mit mindestens 2 C-Atomen,

$R_4$ Alkyl mit 1—4 C-Atomen, Cycloalkyl mit 5—7 C-Atomen, Aralkyl mit 7 oder 8 C-Atomen oder Aryl mit 6—10 C-Atomen,

$R_5$ und $R_6$ unabhängig voneinander Wasserstoff oder Methyl,

$Z_1$ und $Z_3$ je Wasserstoff, $Z_2$ Wasserstoff, Chlor oder Methyl und $Z_4$ Wasserstoff, Methyl, Chlor, —CN, —COOH, —$CONH_2$, Phenyl, Methylphenyl, Methoxyphenyl, Cyclohexyl, Pyridyl, Imidazolyl, Pyrrolidonyl, —COO-Alkyl mit 1—12 C-Atomen im Alkylteil, —COO-Phenyl,

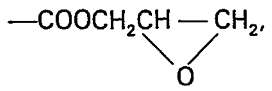

—COO-Alkyl-OH mit 1—3 C-Atomen im Alkyl,

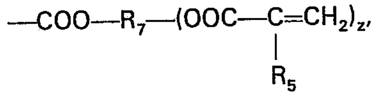

worin $R_7$ einen geradkettigen oder verzweigten gesättigten aliphatischen Rest mit 1—10 C-Atomen, $R_5$ Wasserstoff oder Methyl und z eine genze Zahl von 1—3 bedeuten; —OCO-Alkyl mit 1—4 C-Atomen im Alkyl, —OCO-Phenyl, —CO-Alkyl mit 1—3 C-Atomen im Alkyl, Alkoxy mit 1—6 C-Atomen, Phenoxy, —$CH=CH_2$ oder

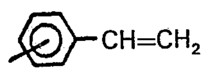

darstellen; oder worin $Z_1$ und $Z_2$ je Wasserstoff und $Z_3$ und $Z_4$ zusammen die Gruppe

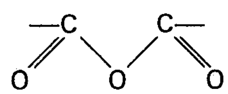

oder je —COOH oder —COO-Alkyl mit 1—6 C-Atomen im Alkyl darstellen.

Stellen $R_1$, $R_2$ oder $R_3$ aliphatische Reste dar, so handelt es sich vor allem um geradkettige oder verzweigte Alkylengruppen mit 2—12 C-Atomen, wobei die Alkylenkette auch durch Heteroatome, wie Sauerstoff, Schwefel- oder Stickstoffatome, unterbrochen sein kann. Bevorzugt handelt es sich dabei um unsubstituierte Alkylengruppen mit 2—10 C-Atomen.

Cycloaliphatische Reste $R_1$ oder $R_2$ sind z.B. die 1,3- oder 1,4-Cyclohexylen-, 1,4-Bis-(methylen)cyclohexan- oder Dicyclohexylmethangruppe, während als araliphatische Reste $R_1$ vor allem 1,3- 1,4- und 2,4-Bis-alkylenbenzol-, 4,4'-Bis-alkylen-diphenyl- und 4,4'-Bis-alkylendiphenyläthergruppen in Betracht kommen.

Carbocyclisch-aromatische und heterocyclisch-aromatische Reste $R_1$ und $R_2$ können auch substituiert sein, z.B. durch Alkyl- oder Alkoxygruppen mit 1—4 C-Atomen oder durch Halogenatome, wie Fluor, Chlor oder Brom.

Carbocyclisch-aromatische Reste $R_1$ und $R_2$ können monocyclisch, kondensiert polycyclisch oder unkondensiert bicyclisch sein, wobei im letzteren Fall die Aromatenkerne bevorzugt über ein Brückenglied miteinander verbunden sind. Bevorzugte carbocyclisch-aromatische Reste $R_1$ sind: die 1,3- und 1,4-Phenylengruppe, der 4,4'-Diphenylmethan-, 4,4'-Diphenyläther- und 4,4'-Diphenylsulfonrest. Als carbocyclischaromatische Reste $R_2$ werden die 1,3- und 1,4-Phenylgruppe, Benzoltriyl- und Benzoltetraylgruppen sowie das Benzophenonringsystem bevorzugt.

Als carbocyclisch-heterocyclische Reste $R_1$ und $R_2$ kommen insbesondere 5- oder 6-gliedrige, O—, N— und/oder S-haltige Ringsysteme in Betracht.

Es können auch Gemische von verschiedenen Polymeren verwendet werden.

Bevorzugt sind a) Polyäther, die wiederkehrende Strukturelemente der Formel X aufweisen, b) Phenol-Formaldehyd-Polykondensate, die wiederkehrende Strukturelemente der Formel XI aufweisen, c) cyclisierte Isoprenpolymere oder Polymere, die gleiche oder unterschiedliche Strukturelemente der Formel XII aufweisen, wie Polyolefine, z.B. Polyäthylen und Polyisopren, Polyvinylchlorid, Polyvinylidenchlorid und deren Copolymere mit anderen Vinylmonomeren, z.B. Vinylacetat, Polyvinylacetat, Styrolpolymere, Acrylpolymere, besonders Polyacrylsäure- und Polymethacrylsäurealkylester, sowie Maleinsäureanhydrid-Polymere.

Besonders bevorzugt sind Polyvinylchlorid, Polystyrol, Polyacrylsäure- und Polymethacrylsäurealkylester mit 1—8 C-Atomen im Alkylteil, cyclisierte Isoprenpolymere, Copolymere von Maleinsäureanhydrid und Vinyläthern oder $\alpha$-Olefinen, wie Methylvinyläther oder Aethylen, sowie Polyäther mit wiederkehrenden Strukturelementen der Formel X.

Als weitere Zusätze können die erfindungsgemässen Stoffgemische z.B. bekannte Härter, Fliessmittel, Haftvermittler und gegebenenfalls auch Triplettsensibilisatoren, wie Phenol, Benzophenon und dergleichen, enthalten.

Die vorerwähnten Polymere können nach an sich bekannten Methoden durch Polykondensation, Polyaddition oder Polymerisation hergestellt werden.

Besonders bevorzugte erfindungsgemäße Stoffgemische sind die folgenden fünf:

1.) Ein Stoffgemisch, das als polymere Verbindung vorzugsweise ein Copolymer aus Maleinsäureanhydrid und Aethylen und als Verbindung der Formel I den 4,4'-Bis-(3-azidophthalsäureimidyl)-diphenyläther enthält, wobei das Gemisch vorzugsweise 50 Gew.%, bezogen auf das Gemisch beider Stoffe, an der polymeren Verbindung enthält.

2.) Ein Stoffgemisch, das als polymere Verbindung vorzugsweise ein Copolymer aus Maleinsäureanhydrid und Methylvinyläther und als Verbindung der Formel I das 4,4'-Bis-(3-azidophthalsäureimidyl)-diphenylmethan enthält, wobei das Gemisch vorzugsweise 75 Gew.%, bezogen auf das Gemisch beider Stoffe, an der polymeren Verbindung enthält.

3.) Ein Stoffgemisch, das als polymere Verbindung vorzugsweise ein Polystyrol und als Verbindung der Formel 1 das 4,4'-Bis-(3-azidophthalsäureimidyl)-diphenylmethan enthält, wobei das Gemisch vorzugsweise 50 Gew.%, bezogen auf das Gemisch beider Stoffe, an der polymeren Verbindung enthält.

4.) Ein Stoffgemisch, das als polymere Verbindung vorzugsweise ein cyclisiertes Polyisopren und als Verbindung der Formel I das 3,3'-Dimethyl-4,4'-bis-(3-azidophthalsäureimidyl)-dicyclohexylmethan enthält, wobei das Gemisch vorzugsweise 35 Gew.%, bezogen auf das Gemisch beider Stoffe, an der polymeren Verbindung enthält.

5.) Ein Stoffgemisch, das als polymere Verbindung vorzugsweise ein Copolymer aus Maleinsäureanhydrid und Methylvinyläther und als Verbindung der Formel I das 1,4-Bis-(3-azidophthalsäureimidyl)-benzol enthält, wobei das Gemisch vorzugsweise 50 Gew.%, bezogen auf das Gemisch beider Stoffe, an der polymeren Verbindung enthält.

Die Bis-Azidophthalsäureimidylderivate der Formel I können dadurch hergestellt werden, dass eine Verbindung der Formel XIII

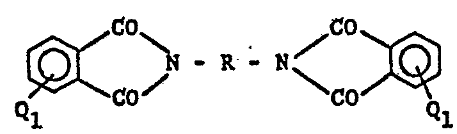

(XIII),

worin

R die unter Formel I angegebene Bedeutung hat und

$Q_1$ ein Halogenatom, wie Chlor, Fluor oder Brom oder, bevorzugt, die Nitrogruppe bedeutet, in einem inerten organischen Lösungsmittel bei einer Temperatur zwischen etwa 30 und 120°C, insbesondere zwischen etwa 70 und 100°C, mit einem Azid der Formel XIV

$$M_1^{n+} (N_3^-)_n \qquad (XIV)$$

umsetzt, worin

n die Zahl 1 oder 2 und

$M_1$ ein Alkalimetall-, Erdalkalimetall- oder quaternäres Ammoniumkation bedeuten.

$Q_1$ stellt bevorzugt die Nitrogruppe dar.

Bedeutet $M_1$ ein quaternäres Ammoniumkation, so handelt es sich z.B. um ein Tetraalkyl- oder ein Benzyltrialkylammoniumkation mit je 1—12 und insbesondere 1—4 C-Atomen in den Alkylteilen, wie das Tetramethyl- und Trimethylbenzylammoniumkation.

Das Azid der Formel XIV wird zweckmässig im Ueberschuss eingesetzt, beispielsweise in einem etwa 5—50%igen und bevorzugt in einem etwa 10—30%igen molaren Ueberschuss.

Vorzugsweise verwendet man Alkalimetallazide, besonders das Natriumazid. Als inerte organische Lösungsmittel für diese Umsetzung eignen sich vor allem polare Lösungsmittel, wie aliphatische Alkohole mit bis zu 6 C-Atomen, Dibenzyl- und Dialkyläther mit je 1—4 C-Atomen in den Alkylteilen, Diäthylenglykol- und Triäthylenglykoldialkyläther mit je 1—4 C-Atomen in den Alkylteilen, aliphatische und aromatische Nitrile, cyclische Amide, N,N-Dialkylamide von aliphatischen Monocarbonsäuren mit 1—3 C-Atomen im Säureteil, Dialkylsulfoxide, wie Dimethyl- und Diäthylsulfoxid, Hexamethylphosphorsäuretriamid und Tetrahydrothiophendioxid.

Die Verbindungen der Formel I können auch dadurch hergestellt werden, dass man eine Verbindung der Formel XV

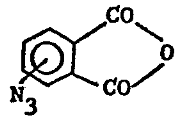

(XV)

mit einem Diamin der Formel XVI

$$H_2N—R—NH_2 \qquad (XVI)$$

zu einer Verbindung der Formel XVII

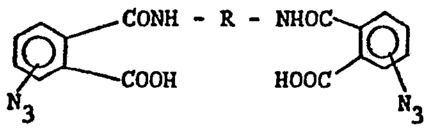

(XVII)

umsetzt, wobei R die unter Formel I angegebene Bedeutung hat, und die Verbindung der Formel XVII anschliessend cyclisiert.

Die Umsetzung der Anhydride der Formel XV mit den Diaminen der Formel XVI wird zweckmässig in organischem Medium vorgenommen, wobei je nach Art der Reaktionskomponenten bei Temperaturen zwischen etwa 0°C und 120°C gearbeitet wird.

Als organische Lösungsmittel kommen vor allem aprotische Lösungsmittel in Betracht, wie gegebenenfalls chlorierte aliphatische oder aromatische Kohlenwasserstoffe, z.B. Benzol, Toluol, Methylenchlorid, Chloroform, Tetrachlorkohlenstoff, 1,2-Dichloräthan und Chlorbenzol; aliphatische und cycloaliphatische Ketone, z.B. Aceton, Methyläthylketon und Cyclohexanon; cyclische Aether, wie Tetrahydrofuran und Dioxan; cyclische Amide, wie N-Methyl-2-pyrrolidon und N-Acetyl-2-pyrrolidon; N,N-Dialkylamide von aliphatischen Monocarbonsäuren mit 1—3 C-Atomen im Säureteil, wie N,N-Dimethylformamid und N,N-Dimethylacetamid, und Dialkylsulfoxide.

Die Cyclisierung der Verbindungen der Formel XVII kann auf an sich bekannte Weise chemisch, d.h. unter Verwendung bekannter Dehydratisierungsmittel, wie Anhydriden von aliphatischen Monocarbonsäuren mit 2—5 C-Atomen, vor allem Essigsäureanhydrid, vorgenommen werden. Je nach Art der Reaktionskomponenten, der Reaktionsbedingungen und des verwendeten Lösungsmittels, kann die Cyclisierung, besonders bei erhöhten Temperaturen, auch ohne Zusatz eines Dehydratisierungsmittels erfolgen, wobei das gebildete Wasser zweckmässig azeotrop entfernt wird.

Die Verbindungen der Formeln XIII bis XVI sind bekannt oder können auf an sich bekannte Weise hergestellt werden. Verbindungen der Formel XIII können z.B. durch Umsetzen von 3- oder 4-Nitrophthalsäureanhydrid oder den entsprechenden Halogenverbindungen mit Diaminen der Formel XVI und anschliessende Cyclisierung der dabei entstehenden Amidcarbonsäuren erhalten werden.

Die erfindungsgemässen Stoffgemische lassen sich unter der Einwirkung von Licht, besonders UV-Licht, härten bzw. vernetzen und eignen sich beispielsweise zur Herstellung von Druckplatten für das Offsetdruckverfahren, zur Herstellung von Photooffset-Lacken, für die unkonventionnelle Photographie, z.B. zur Herstellung von sogenannten Vesikularbildern oder zum Anfärben von nach dem Belichten und Entwickeln schlecht sichtbaren Polymerbildern mit geeigneten Farbstoffen, wie öllösliche Farbstoffe oder, wenn das Polymere saure Gruppen, wie Carbonsäure- oder Sulfonsäuregruppen aufweist, kationische Farbstoffe. Die erfindungsgemässen Stoffgemische finden insbesondere Anwendung als sogenannte Photoresists zur Herstellung von gedruckten Schaltungen nach an sich bekannten Methoden. Dabei wird die mit der lichtempfindlichen Schicht versehene Seite der Leiterplatte durch ein das Lieterbild aufweisendes Dianegativ belichtet und dann entwickelt, worauf man die unbelichteten Stellen der Schicht durch Entwicklungsflüssigkeit herausholt.

Die Belichtung kann mit Sonnenlicht, Kohlelichtbogen oder Xenonlampen durchgeführt werden. Mit Vorteil wird die Belichtung mit Quecksilberhochdrucklampen vorgenommen.

Die Trägermaterialien können nach an sich üblichen Techniken mit dem lichtempfindlichen Stoffgemisch beschichtet werden, z.B. durch Tauchverfahren, Sprüh-, Schleuder-, Kaskaden- und Vorhangguss, oder mit Hilfe einer Walze.

Die erfindungsgemässen Stoffgemische werden im allgemeinen in Form von Lösungen oder Suspensionen in geeigneten organischen Lösungsmitteln oder Lösungsmittelgemischen, wie N,N-Dialkylamiden von aliphatischen Monocarbonsäuren, aliphatischen oder cyclischen Ketonen oder cyclischen Aethern der vorerwähnten Art, eingesetzt.

## Beispiel 1

Mit einer Lösung von 3 g 1,4-Bis-(3-Azidophthalimidyl)-butan und 5 g eines Copolymeren aus Maleinsäureanhydrid und Aethylen ("EMA 21"®, Handelsprodukt der Fa. Monsanto) in 72 ml N,N-Dimethylformamid wird eine Aluminiumplatte mittels einer Schleuderzentrifuge beschichtet (300 Umdrehungen/Minute). Nach dem Trocknen des Ueberzuges wird die beschichtete Platte durch ein Strichnegativ während 1 Minute mit UV-Licht belichtet (Quecksilber-Hochdruckbrenner mit vorangeschaltetem Pyrex-Filter, Wellenlänge über 320 nm). Danach wird die belichtete Platte in 5- bis 10%iger wässriger Natriumbicarbonat-Lösung entwickelt, wobei ein dem Stichnegativ entsphrechendes Reliefbild entsteht, das gegebenenfalls mit einem kationischen Farbstoff angefärbt werden kann.

Beispiele 2 bis 13

In der folgenden Tabelle sind weitere erfindungsgemässe Stoffgemische angeführt, mit denen wie in Beispiel 1 beschrieben Abbildungen auf photoempfindliche Platten hergestellt werden können.

TABELLE

| Beispiel | Zusammensetzung | | Gewichtsprozent | |
|---|---|---|---|---|
| | Bis-Azid der Formel I | Polymer | Azid | Polymer |
| 2 | 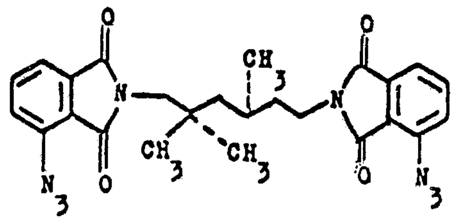 , (Diaminkomponente = Isomerengenisch von 1,6-Diamino-2-methyl-4-dimethylhexan und 1,6-Diamino-2-dimethyl-4-methylhexan) | 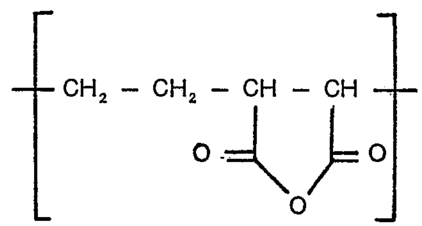 | 37,5 | 62,5 |
| 3 | 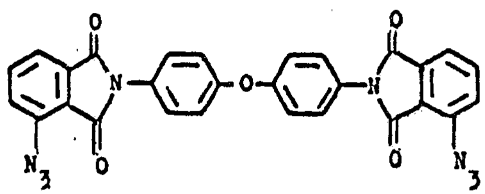 | do. | 50 | 50 |
| 4 | 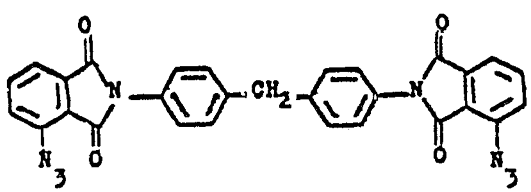 | do. | 33,5 | 66,5 |

TABELLE (Fortsetzung)

| Beispiel | Zusammensetzung | | Gewichtsprozent | |
|---|---|---|---|---|
| | Bis-Azid der Formel I | Polymer | Azid | Polymer |
| 5 | 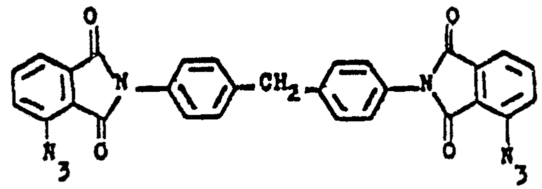 | 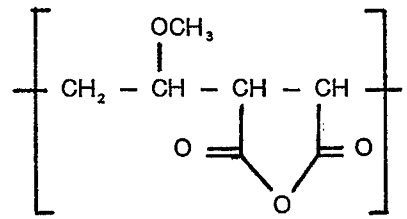 (Gantrez 119)* | 26 | 74 |
| 6 | 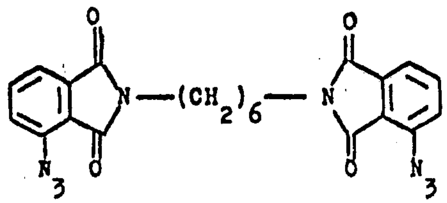 | 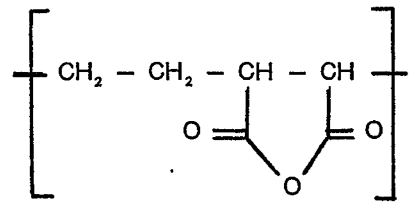 | 50 | 50 |
| 7 | 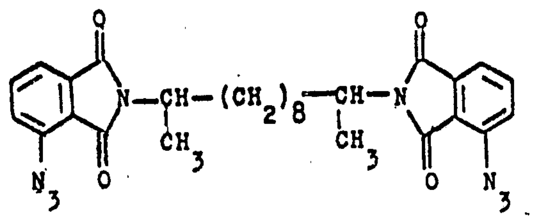 (Diaminkomponente gemäss DT–OS 2.549.403 | do. | 50 | 50 |
| 8 | 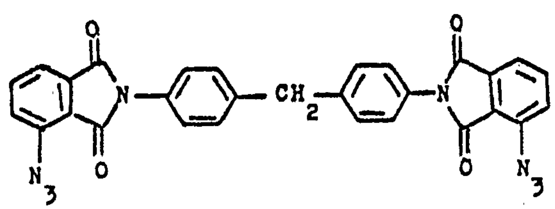 | 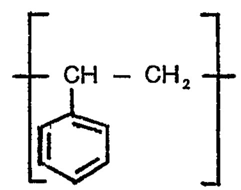 | 50 | 50 |

*) "Gantrez 119 AN", ® Handelsprodukt der Fa. General Aniline & Film Corporation.

TABELLE (Fortsetzung)

| Beispiel | Zusammensetzung | | Gewichtsprozent | |
|---|---|---|---|---|
| | Bis-Azid der Formel I | Polymer | Azid | Polymer |
| 9 | 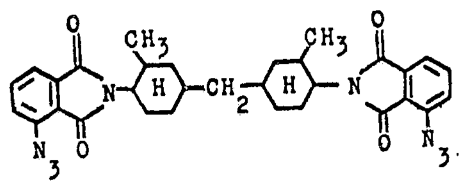 | Isopren Typ I* J. Pol. Scie. A–1 (Vol 10) 1839–1850 (1972) | 64,94 | 35 |
| 10 | 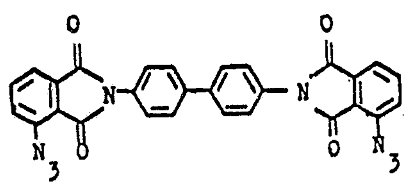 | 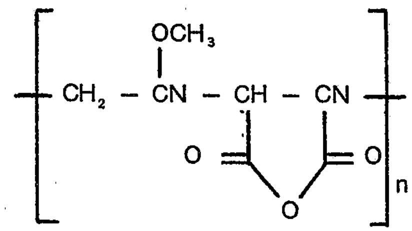 | 50 | 50 |
| 11 | 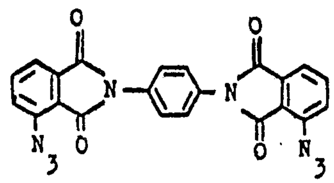 | 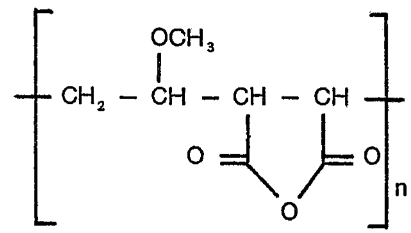 | 50 | 50 |

TABELLE (Fortsetzung)

| Beispiel | Zusammensetzung | | Gewichtsprozent | |
|---|---|---|---|---|
| | Bis-Azid der Formel I | Polymer | Azid | Polymer |
| 12 | 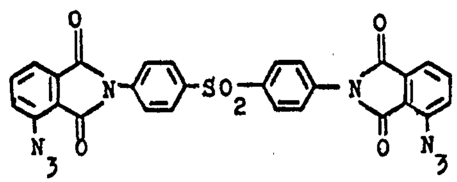 | 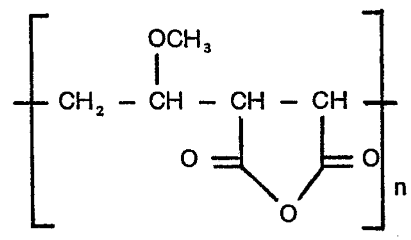 | 50 | 50 |
| 13 | 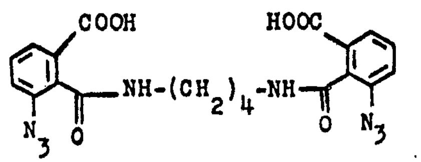 | 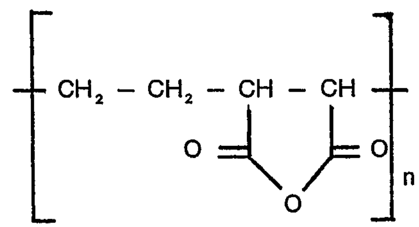 | 40 | 60 |

Beispiel 14

1,35 g 4,4'-Bis-(3-Azidophthalimidyl)-diphenyläther werden in 100 m Cyclohexan gelöst und dann unter Lichtausschluss mit 100 g einer 2,5%igen Lösung eines synthetischen Isopren-Polymeren ("Cariflex IR 309"®, Handelsprodukt der Fa. Shell AG) in Chlorbenzol vermischt.

Mit dieser Lösung wird eine kupferkaschierte Epoxiplatte beschichtet, die Schicht wird bei 40°C im Vakuum getrocknet und dann durch ein Negativ mit UV-Licht ($\lambda$ über 320 nm) belichtet. Nach dem Entwickeln mit 1,1,1-Trichloräthan erschein ein dem Negativ entsprechendes Bild.

Beispiel 15

Unter Lichtausschluss werden 4,0 g 4,4'-Bis-(3-Azidophthalimidyl)-diphenyläther in 27 ml Cyclohexanon gelöst und anschliessend mit 6,0 g eines Gemisches, bestehend aus 99,5 Gewichtsteilen eines Epoxidharzes (Umsetzungsprodukt von 72,82 Gewichtsteilen Epichlorhydrin und 27,18 Gewichtsteilen 2,2-Bis-(p-hydroxyphenyl)-propan, Epoxidgehalt 1,2 bis 1,4 Epoxidäquivalente/kg), 4,8 Gewichtsteilen eines Härters (o-Tolylbiguanid aus 59,4 Gewichtsteilen o-Toluidin und 40,6 Gewichtsteilen Dicyandiamid) und 0,5 Gewichtsteilen eines die Fliess- und Adhäsionseigenschaften der Beschichtungsmasse verbessernden Mittels ("Modaflow"®, Handelsprodukt der Fa. Monsanto) in 16 ml N,N-Dimethylformamid vermischt. Mit der erhaltenen Lösung wird eine kupferkaschierte Epoxiplatte beschichtet und die Schicht wird bei 40°C im Vakuum getrocknet und dann durch ein Negativ mit UV-Licht ($\lambda$ über 320 nm) belichtet. Nach dem Entwickeln mit 1,1,1-Trichloräthan erhält man ein dem Negativ entsprechendes Bild.

Die in den vorangehenden Beispielen verwendeten Bis-Azidophthalimide können wie folgt hergestellt werden:

a) *1,4-Bis-(3-Azidophthalimidyl)-butan*

Ein Gemisch von 21,3 g (0,048 Mol) 1,4-Bis-(3-Nitrophthalimidyl)-butan und 7,2 g (0,11 Mol) Natriumazid in 225 ml N,N-Dimethylformamid wird 18 Stunden auf 80°C erhitzt und nachher bei der gleichen Temperatur im Vakuum eingedampft. Der Rückstand wird mit 200 ml Wasser verrührt und mit 1 ml konz. Salzsäure angesäuert. Die entstandene Suspension des Produktes wird abgesaugt, mit 20 ml Wasser nachgewaschen und 24 Stunden bei 80°C im Trockenschrank getrocknet. Man erhält 20,4 g (98,8% d.Th.) 1,4-Bis-(3-Azidophthalimidyl)-butan; Fp. 162°C (Zersetzung).

Das 1,4-Bis-(3-Nitrophthalimidyl)-butan kann wie folgt hergestellt werden: In einem Autoklaven werden 8,1 g (0,042 Mol) 3-Nitrophthalsäureanhydrid in 15 ml Essigsäure suspendiert, mit 1,76 g (0,02 Mol) 1,4-Diaminobutan in 15 ml Toluol versetzt und 6 Stunden bei 120°C gerührt. Der entstandene dicke Niederschlag wird abgesaugt und 24 Stunden bei 120°C/100 Torr getrocknet. Man erhält 7,8 g (90% d.Th.) 1,4-Bis-(3-Nitrophthalimidyl)-butan; Fp. 245°C.

b) *Isomerengemisch von 1,6-Bis-(3-Azidophthalimidyl)-2-methyl-4-dimethylhexan und 1,6-Bis-(3-Azidophthalimidyl)-2-dimethyl-4-methylhexan.*

Die Herstellung erfolgt analog dem unter a) beschriebenen Verfahren unter Verwendung von 20,3 g (0,04 Mol) eines Isomeren gemisches von 1,6-Bis-(3-Nitrophthalimidyl)-2-methyl-4-dimethylhexan und -2-dimethyl-4-methylhexan und 7,78 g (0,104 Mol) Natriumazid. Man erhält ein Isomerengemisch in Form einer feinverteilten glasigen Masse, die beim Erhitzen zerfliesst und sich bei 180°C unter Aufschäumen zersetzt.

c) *4,4'-Bis-(3-Azidophthalimidyl)-diphenylmethan*

Die Herstellung erfolgt analog dem unter a) beschriebenen Verfahren unter Verwendung von 9,5 (0,017 Mol) 4,4'-Bis-(3-Nitrophthalimidyl)-diphenylmethan und 2,8 g (0,044 Mol) Natriumazid. Ausbeute 81,5% d.Th.: Fp 182°C (Zersetzung).

d) *4,4'-Bis-(3-Azidophthalimidyl)-diphenyläther*

Die Herstellung erfolgt analog dem unter a) beschriebenen Verfahren unter Verwendung von 22 g (0,04 Mol) 4,4'-Bis-(3-Nitrophthalimidyl)-diphenyläther und 7,78 (0,104 Mol) Natriumazid. Ausbeute 88,5% d.Th.; Fp. 185°C (Zersetzung).

e) *1,6-Bis-(3-Azidophthalimidyl)-hexan*

Die Herstellung erfolgt analog dem unter a) beschriebenen Verfahren unter Verwendung von 20 g (0,043 Mol) 1,6-Bis-(3-Nitrophthalimidyl)-hexan und 7,2 g (0,111 Mol) Natriumazid. Ausbeute 78% d.Th.; Fp. 147°C (Zersetzung).

f) *1,10-Bis-(3-Azidophthalimidyl)-1,10-dimethyldecan*

Die Herstellung erfolgt analog dem unter a) beschriebenen Verfahren unter Verwendung von 8 g (0,014 Mol) 1,10-Bis-(3-Nitrophthalimidyl)-1,10-dimethyldecan und 2,36 g (0,035 Mol) Natriumazid. Ausbeute 80% d.Th. Das Reaktionsprodukte fällt in Form eines dickflüssigen Oels an.

**Patentansprüche**

1. Ein unter der Einwirkung von Licht härtbares Stoffgemisch, das mindestens eine gegebenenfalls härtbare polymere Verbindung und mindestens eine Verbindung der Formel I

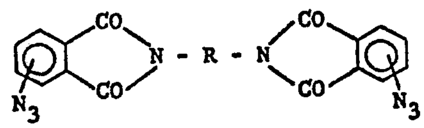

(I)

enthält, worin

R unsubstituiertes oder substituiertes Alkylen mit 2 bis 12 C-Atomen, unsubstituiertes oder substituiertes Phenylen, Naphthylen, Biphenylen, Cyclohexylen, Dicyclohexylmethan oder ein unsubstituierte oder substituierte Gruppe

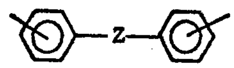

und

Z, —O—, —S—, $—SO_2—$, $—CH_2—$, —CO—, $—CH(CH_3)—$ oder $—C(CH_3)_2—$

bedeuten, wobei das Gewichtsverhältnis von polymerer Verbindung zu Verbindung der Formel I 9:1 bis 1:4 beträgt.

2. Ein Stoffgemisch nach Anspruch 1, worin das Gewichtsverhältnis von polymerer Verbindung zu Verbindung der Formel I 9:1 bis 1:1 beträgt.

3. Ein Stoffgemisch nach Anspruch 1, in dem in der Verbindung der Formel I die $N_3$-Gruppen je in 3-Stellung an den Benzolring gebunden sind.

4. Ein Stoffgemisch nach Anspruch 1, in dem R in Formel I unsubstituiertes oder durch eine oder zwei Phenylgruppen, Cycloalkylgruppen mit 5 bis 8 C-Atomen oder Aralkylgruppen mit 7 oder 8 C-Atomen substituiertes Alkylen, unsubstituiertes oder pro Ring durch eine Alkylgruppe mit 1 bis 4 C-Atomen, eine —OH—, $—CO^-M^+$- oder $—SO_3^-M^+$-Gruppe substituiertes Phenylen, Naphthylen, Biphenylen, Cyclohexylen, Dicyclohexylmethan oder

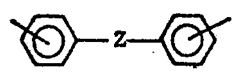

darstellt, wobei Z die unter Formel I angegebene Bedeutung hat, $M^+$ ein Wasserstoffion, ein Alkalimetallkation, das Pyridiniumkation oder die Gruppe

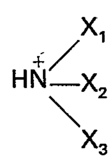

bedeutet, in der $X_1$ und $X_2$ unabhängig voneinander Wasserstoff oder Alkyl mit 1 bis 12 C-Atomen und $X_3$ gleich Wasserstoff, Alkyl mit 1 bis 12 C-Atomen oder Benzyl sind.

5. Ein Stoffgemisch nach Anspruch 1, worin die polymere Verbindung ein Polyester, ein Polyesteramid, ein Polyamid, eine Polyamidsäure, eine Polyamid-amidsäure, ein Polyimid, ein Polyamid-imid, ein Polyäther, ein Polyamin, ein Polyimin, ein Polyurethan, ein Polyharnstoff, ein Polyurethan-harnstoff, eine Polycarbonat, ein Phenol-Formaldehyd-Polykondensat, eine Polysaccharid, Gelatine oder ein Polymeres ist, das durch Homo- oder Copolymerisation von reaktive C=C-Doppelbindungen enthaltenden Monomeren erhalten wird.

6. Ein Stoffgemisch nach Anspruch 5, worin a) der Polyäther wiederkehrende Strukturelemente der Formel X aufweist

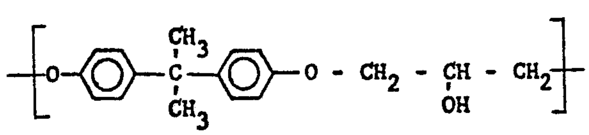

(X),

b) das Phenol-Formaldehyd-Polykondensat wiederkehrende Strukturelemente der Formel XI aufweist

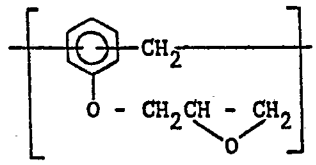

(XI),

c) das Polymere ein cyclyciertes Isoprenpolymeres ist oder gleiche oder verschiedene wiederkehrende Strukturelemente der Formel XII aufweist

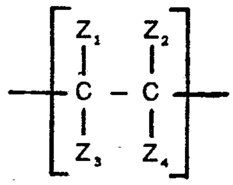

(XII),

worin $Z_1$ und $Z_3$ je Wasserstoff, $Z_2$ Wasserstoff, Chlor oder Methyl und $Z_4$ Wasserstoff, Methyl, Chlor, —CN, —COOH, $—CONH_2$, Phenyl, Methylphenyl, Methoxyphenyl, Cyclohexyl, Pyridyl, Imidazolyl, Pyrrolidonyl, —COO-Alkyl mit 1 bis 12 C-Atomen im Alkylteil, —COO-Phenyl,

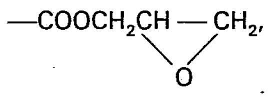

—COO-Alkyl-OH mit 1 bis 3 C-Atomen im Alkyl,

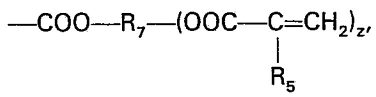

worin $R_7$ einen geradkettigen oder verzweigten gesättigten aliphatischen Rest mit 1 bis 10 C-Atomen, $R_5$ Wasserstoff oder Methyl und z eine genze Zahl von 1 bis 3 bedeuten; —OCO-Alkyl mit 1 bis 4 C-Atomen im Alkyl, —OCO-Phenyl, —CO-Alkyl mit 1 bis 3 C-Atomen im Alkyl, Alkoxy mit 1 bis 6 C-Atomen, Phenoxy, $—CH=CH_2$ oder

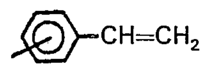

darstellen; oder worin $Z_1$ und $Z_2$ je Wasserstoff und $Z_3$ und $Z_4$ zusammen die Gruppe

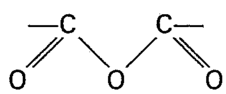

oder je —COOH oder —COO-Alkyl mit 1—6 C-Atomen im Alkyl darstellen.

7. Ein Stoffgemisch nach Anspruch 6, worin das Polymere ein Polyvinylchlorid, ein Polystyrol, ein Polyacrylsäure- oder Polymethacrylsäurealkylester mit 1 bis 8 C-Atomen im Alkylteil oder ein Copolymeres von Maleinsäureanhydrid und Methylvinyläther oder Aethylen ist.

8. Ein Stoffgemisch nach Anspruch 1 oder 7, das als polymere Verbindung ein Copolymer aus Maleinsäureanhydrid und Aethylen und als Verbindung der Formel I den 4,4′-Bis-(3-azidophthalsäureimidyl)-diphenyläther enthält, wobei das Gemisch vorzugsweise 50 Gew.%, bezogen auf das Gemisch beider Stoffe, an der polymeren Verbindung enthält.

9. Ein Stoffgemisch nach Anspruch 1 oder 7, das als polymere Verbindung ein Copolymer aus Maleinsäureanhydrid und Methylvinyläther und als Verbindung der Formel I das 4,4′-Bis-(3-azidophthalsäureimidyl)-diphenylmethan enthält, wobei das Gemisch vorzugsweise 75 Gew.%, bezogen auf das Gemisch beider Stoffe, an der polymeren Verbindung enthält.

10. Ein Stoffgemisch nach Anspruch 1 oder 7, das als polymere Verbindung ein Polystyrol und als Verbindung der Formel I das 4,4′-Bis-(3-azidophthalsäureimidyl)-diphenylmethan enthält, wobei das Gemisch vorzugsweise 50 Gew.-%, bezogen auf das Gemisch beider Stoffe, an der polymeren Verbindung enthält.

11. Ein Stoffgemisch nach Anspruch 1 oder 6, das als polymere Verbindung ein cyclisiertes

Isoprepolymeres und als Verbindung der Formel I das 3,3'-Dimethyl-4,4'-bis-(3-azidophthalsäureimidyl)-dicyclohexylmethan enthält, wobei das Gemisch vorzugsweise 35 Gew.%, bezogen auf das Gemisch beider Stoffe, an der polymeren Verbindung enthält.

12. Ein Stoffgemisch nach Anspruch 1 oder 7, das als polymere Verbindung ein Copolymer aus Maleinsäureanhydrid und Methylvinyläther und als Verbindung der Formel I das 1,4-Bis-(3-azidophthalsäureimidyl)-benzol enthält, wobei das Gemisch vorzugsweise 50 Gew.%, bezogen auf das Gemisch beider Stoffe, an der polymeren Verbindung enthält.

13. Verwendung eines Stoffgemisches nach Anspruch 1 zur Vernetzung unter der Einwirkung von Licht, besonders zum Erzeugen von Abbildungen.

**Revendications**

1. Mélange durcissable sous l'action de la lumière, mélange qui contient au moins un composé polymère éventuellement durcissable et au moins un composé répondant à la formule I

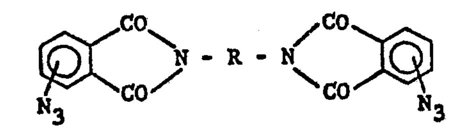

(I)

dans laquelle

R représente un radical alkylène contenant de 2 à 12 atomes de carbone, substitué ou non, un radical dicyclohexylméthane, cyclohexylène, biphénylylène, naphtylène ou phénylène, substitué ou non, ou un groupement, substitué ou non, répondant à la formule

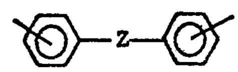

dans laquelle Z représente —O—, —S—, $—SO_2—$, $—CH_2—$, —CO—, $—CH(CH_3)—$ ou $—C(CH_3)_2—$

le rapport pondéral du composé polymère au composé de formule I étant compris entre 9:1 et 1:4.

2. Mélange selon la revendication 1 dans lequel le rapport pondéral du composé polymère au composé de formule I est compris entre 9:1 et 1:1.

3. Mélange selon la revendication 1 caractérisé en ce que, dans le composé de formule I, les groupes $—N_3$ se trouvent chacun en position 3 sur le noyau benzénique correspondant.

4. Mélange selon la revendication 1 caractérisé en ce que, dans la formule I, R représente un radical alkylène non substitué ou porteur d'un ou deux substituants pris dans l'ensemble constitué par les radicaux phényles, les radicaux cycloalkyles contenant de 5 à 8 atomes de carbone et les radicaux aralkyles contenant 7 ou 8 atomes de carbone, ou un radical phénylène, naphtylène, biphénylylène, cyclohexylène, dicyclohexylméthane ou

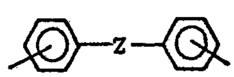

non substitué ou portant, par noyau, un radical alkyle en $C_1—C_4$ ou un groupe —OH, $—COO^-M^+—$ ou $—SO_3^-M^+$, le symbole Z ayant la signification indiquée à propos de la formule I et $M^+$ représentant un ion d'hydrogène, un cation de métal alcalin, un cation pyridinium ou un groupe

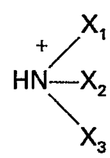

dans laquel $X_1$ et $X_2$ représentent chacun, indépendamment l'un de l'autre, l'hydrogène ou un alkyle en $C_1—C_{12}$ et $X_3$ représente l'hydrogène, un alkyle en $C_1—C_{12}$ ou un benzyle.

5. Mélange selon la revendication 1 dans lequel le composé polymère est un polyester, un polyesteramide, un polyamide, un polyamide-acide, un polyamide-amide-acide, un poly-imide, un polyamide-imide, un polyéther, une polyamine, une polyimine, un polyuréthanne, une poly-urée, une

poly-uréthanne-urée, un polycarbonate, un produit de polycondensation phénol-formaldéhyde, un polysaccharide, la gélatine ou un polymère qui a été obtenu par homopolymérisation ou copolymérisation de monomères contenant des doubles liaisons C=C réactives.

6. Mélange selon la revendication 5 dans lequel:

a) le polyéther comporte des motifs de formule X

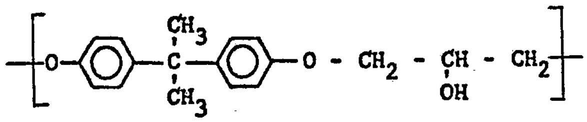

(X),

b) le produit de polycondensation phénol-formaldéhyde comporte des motifs de formule XI

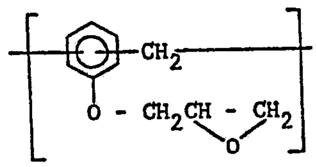

(XI),

c) le polymère est un polymère de l'isoprène cyclisé ou comporte des motifs, identiques ou différents, répondant à la formule XII

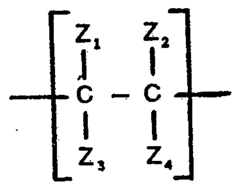

(XII)

dans laquelle

$Z_1$ et $Z_3$ représentent chacun l'hydrogène,

$Z_2$ représente l'hydrogène, le chlore ou un méthyle et

$Z_4$ représente l'hydrogène, un méthyle, le chlore, —CH, —COOH, —$CONH_2$, phényle, méthylphényle, méthoxyphényle, cyclohexyle, pyridyle, imidazolyle, pyrrolidonyle, —COO-alkyle à alkyle en $C_1$—$C_{12}$, —COO-phényle,

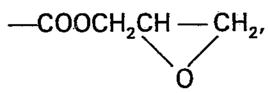

—COO-Alkyl-OH à alkyle en $C_1$—$C_3$, un radical

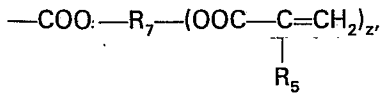

dans lequel $R_7$ représente un radical aliphatique saturé, linéaire ou ramifié, contenant de 1 à 10 atomes de carbone, $R_5$ représente l'hydrogène ou un méthyle et z un nombre entier de 1 à 3; un radical —OCO-alkyle dont l'alkyle contient de 1 à 4 atomes de carbone, —OCO-phènyle, —CO-alkyle à alkyle en $C_1$—$C_3$, alcoxy en $C_1$—$C_6$, phénoxy, —$CH{=}CH_2$ ou

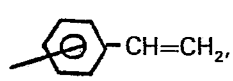

ou dans laquelle

$Z_1$ et $Z_2$ représentent chacun l'hydrogène et

$Z_3$ et $Z_4$ forment ensemble un groupement

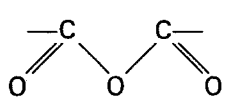

ou représentent chacun —COOH ou un radical —COO-alkyle contenant de 1 à 6 atomes de carbone dans la partie alkylique.

7. Mélange selon la revendication 6 caractérisé en ce que le polymère est un polychlorure de vinyle, un polystyrène, un polyacrylate ou polyméthacrylate d'alkyle contenant de 1 à 8 atomes de carbone dans la partie alkylique, ou un copolymère de l'anhydride maléique et de l'oxyde de méthyle et de vinyle ou de l'éthylène.

8. Mélange selon l'une des revendications 1 et 7 qui contient, comme composé polymère, un copolymère de l'anhydride maléique et de l'éthylène et, comme composé de formule I, l'oxyde de bis-[(azido-3 phtalimidyl)-4 phényle], la proportion du composé polymère étant de préférence de 50% en poids par rapport au mélange des deux corps.

9. Mélange selon l'une des revendications 1 et 7 qui contient, comme composé polymère, un copolymère de l'anhydride maléique et de l'oxyde de méthyle et de vinyle et, comme composé de formule I, le bis-(azido-3 phtalimidyl)-4,4' diphénylméthane, la proportion du composé polymère étant de préférence de 75% en poids par rapport au mélange des deux corps.

10. Mélange selon l'une des revendications 1 et 7 qui contient, comme composé polymère, un polystyrène et, comme composé de formule I, le bis-(azido-3 phtalimidyl)-4,4' diphénylméthane, la proportion du composé polymère étant de préférence de 50% en poids par rapport au mélange des deux corps.

11. Mélange selon des revendications 1 et 6 qui contient, comme composé polymère, un polymère de l'isoprène cyclique et, comme composé de formule I, le diméthyl-3,3' bis-(azido-3 phtalimididyl)-4,4' dicyclohexylméthane, la proportion du composé polymère étant de préférence de 35% en poids par rapport au mélange des deux corps.

12. Mélange selon l'une des revendications 1 et 7 qui contient, comme composé polymère, un copolymère de l'anhydride maléique et de l'oxyde de méthyle et de vinyle et, comme composé de formule I, le bis-(azido-3 phtalimidyl)-1,4 benzène, la proportion du composé polymère étant de préférence de 50% en poids par rapport au mélange des deux corps.

13. Utilisation d'un mélange selon la revendication 1 pour la réticulation sous l'action de la lumière, en particulier pour la production d'images.

## Claims

1. A photo-curable composition of matter which contains at least one optionally curable polymeric compound and at least one compound of the formula I

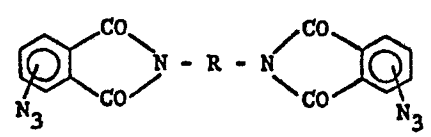

(I)

in which R is unsubstituted or substituted alkylene having 2 to 12 C atoms, unsubstituted or substituted phenylene, naphthylene, biphenylene, cyclohexylene or dicyclohexylmethane or an unsubstituted or substituted

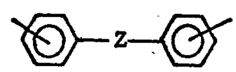

group and Z is —O—, —S—, $—SO_2—$, $—CH_2—$, —CO—, $—CH(CH_3)—$ or $—C(CH_3)_2—$,

the weight ratio of the polymeric compound to the compound of the formula I being 9:1 to 1:4.

2. A composition of matter according to claim 1, wherein the weight ratio of the polymeric compound to the compound of the formula I is 9:1 to 1:1.

3. A composition of matter according to claim 1, in which the $N_3$ groups in the compound of the formula I are each bonded to the benzene ring in the 3-position.

4. A composition of matter according to claim 1, in which R in formula I is alkylene which is unsubstituted or substituted by one or two phenyl groups, cycloalkyl groups having 5 to 8 C atoms or aralkyl groups having 7 or 8 C atoms, or phenylene, naphthylene, biphenylene, cyclohexylene, dicyclohexylmethane or

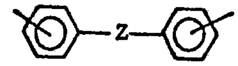

which are unsubstituted or substituted, per ring, by one alkyl group having 1 to 4 C atoms, one —OH, $—COO^-M^+$ or $—SO_3^-M^+$ group, Z is as defined under formula I, $M^+$ is a hydrogen ion, an alkali metal cation, the pyridinium cation or the

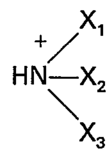

group, wherein $X_1$ and $X_2$ independently of one another are hydrogen or alkyl having 1 to 12 C atoms and $X_3$ is hydrogen, alkyl having 1 to 12 C atoms or benzyl.

5. A composition of matter according to claim 1, in which the polymeric compound is a polyester, a polyesteramide, a polyamide, a polyamidoacid, a polyamide-amido-acid, a polyimide, a polyamidoimide, a polyether, a polyamine, a polyimine, a polyurethane, a polyurea, a polyurethane-urea, a polycarbonate, a phenol-formaldehyde polycondensate, a polysaccharide, gelatin or a polymer which is obtained by homopolymerisation or copolymerisation of monomers containing reactive C=C double bonds.

6. A composition of matter according to claim 5, wherein a) the polyether contains recurring structural units of the formula X

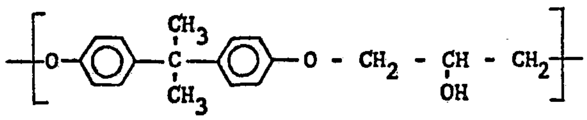

(X)

b) the phenol-formaldehyde polycondensate contains recurring structural units of the formula XI

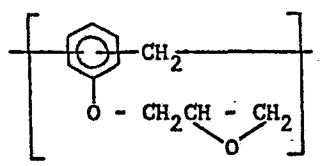

(XI)

c) the polymer is a cyclised isoprene polymer or contains identical or different recurring structural units of the formula XII

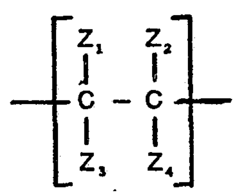

(XII);

wherein each of $Z_1$ and $Z_3$ is hydrogen, $Z_2$ is hydrogen, chlorine or methyl and $Z_4$ is hydrogen, methyl, chlorine, —CN, —COOH, $—CONH_2$, phenyl, methylphenyl, methoxyphenyl, cyclohexyl, pyridyl, imidazolyl, pyrrolidonyl, —COO-alkyl having 1 to 12 C atoms in the alkyl moiety, —COO-phenyl,

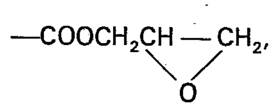

—COO-Alkyl-OH having 1 to 3 C atoms in the alkyl,

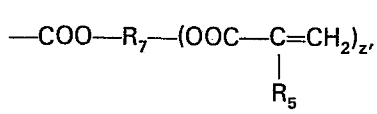

in which $R_7$ is a straight-chain or branched saturated aliphatic radical having 1 to 10 C atoms, $R_5$ is hydrogen or methyl and z is an integer from 1 to 3, or —OCO-alkyl having 1 to 4 C atoms in the alkyl, —OCO-phenyl, —CO-alkyl having 1 to 3 C atoms in the alkyl, alkoxy having 1 to 6 C atoms, phenoxy, $—CH=CH_2$ or

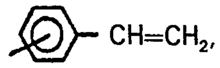

or each of $Z_1$ and $Z_2$ is hydrogen, and $Z_3$ and $Z_4$ together are the

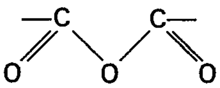

group or each is —COOH or —COO-alkyl having 1 to 6 C atoms in the alkyl moiety.

7. A composition of matter according to claim 6, wherein the polymer is a polyvinyl chloride, a polystyrene, a poly(alkyl acrylate) or poly(alkyl methacrylate) having 1 to 8 C atoms in the alkyl moiety, or a copolymer of maleic anhydride and methyl vinyl ether or ethylene.

8. A composition of matter according to either of claims 1 or 7, which contains a copolymer of maleic anhydride and ethylene as the polymeric compound and 4,4′-bis-(3-azidophthalimidyl)-diphenyl ether as the compound of the formula I, the mixture preferably containing 50% by weight, based on the mixture of the two substances, of the polymeric compound.

9. A composition of matter according to either of claims 1 or 7, which contains a copolymer of maleic anhydride and methyl vinyl ether as the polymeric compound and 4,4′-bis-(3-azidophthalimidyl)-diphenylmethane as the compound of the formula I, the mixture preferably containing 75% by weight, based on the mixture of the two substances, of the polymeric compound.

10. A composition of matter according to either of claims 1 or 7, which preferably contains a polystyrene as the polymeric compound and 4,4′-bis-(3-azidophthalimidyl)-diphenylmethane as the compound of the formula I, the mixture preferably containing 50% by weight, based on the mixture of the two substances, of the polymeric compound.

11. A composition of matter according to either of claims 1 or 6, which contains a cyclised isoprene polymer as the polymeric compound and 3,3′-dimethyl-4,4′-bis-(3-azidophthalimidyl)-dicyclohexylmethane as the compound of the formula I, the mixture preferably containing 35% by weight, based on the mixture of the two substances, of the polymeric compound.

12. A composition of matter according to either of claims 1 or 7, which contains a copolymer of maleic anhydride and methyl vinyl ether as the polymeric compound and 1,4-bis-(3-azidophthalimidyl)-benzene as the compound of the formula I, the mixture preferably containing 50% by weight, based on the mixture of the two substances, of the polymeric compound.

13. The use of a composition of matter according to claim 1 for photo-crosslinking, especially for making reproductions.